# EUROPÄISCHE PATENTANMELDUNG

(11) **EP 0 674 468 A2**
(43) Veröffentlichungstag der Anmeldung: **27.09.1995**
(21) Anmeldenummer: 95104222.5
(22) Anmeldetag: 22.03.1995
(51) Int. Cl.: H05B 33/08, H05B 39/09

(54) **Farblicht-Behandlungsgerät**

(30) Priorität: 24.03.1994 DE 9405071 U; 03.11.1994 DE 9417441 U
(71) Anmelder: SYNTON GmbH FORSCHUNG-ENTWICKLUNG-VERTRIEB, D-94469 Deggendorf (DE)
(72) Erfinder: Lenke, Michael, D-94526 Metten (DE)
(74) Vertreter: KUHNEN, WACKER & PARTNER

(57) **Zusammenfassung**

Die Erfindung betrifft ein Farblicht-Behandlungsgerät mit einer Farblicht-Strahlungsquelle. Die Farblicht-Strahlungsquelle ist mittels einer Steuereinrichtung in einen Blinklichtbetrieb mit einer bestimmten Blinkfrequenz versetzbar, wobei zur Steuerung der Blinkfrequenz ein Sensor zur Erfassung eines sich zeitlich verändernden Körperparameters des Benutzers oder Anwenders des Farblicht-Behandlungsgeräts vorhanden sein kann.

## Beschreibung

Die Erfindung betrifft ein Farblicht-Behandlungsgerät mit einer ein- oder vorzugsweise mehrfarbigen Farblicht-Strahlungsquelle.

Es sind Farblicht-Behandlungsgeräte bekannt, die mit Farblichtquellen in Form von Leuchtdioden arbeiten, die matrixförmig angeordnet sind und Farblicht mit schmalem Wellenlängenbereich erzeugen. Hierbei können Leuchtdiodenfelder entweder nur mit einfarbigen oder aber mit einer Mischung aus verschiedenfarbigen Leuchtdioden bestückt sein.

Allgemein übt eine Farblichtbehandlung verschiedene positive Wirkungen auf die Zellen des Körpers des Anwenders aus und kann die Gewebe-Elastizität anregen, den Lymphfluß aktivieren, den Hautstoffwechsel fördern, die Durchblutung anregen usw. Auch kann die Farblichtbehandlung bei der Schmerzbekämpfung wirksam sein.

Das erfindungsgemäße Farblicht-Behandlungsgerät ermöglicht zusätzlich eine Blinklichtbehandlung des Anwenders, bei der das Behandlungslicht intermittierend zum Aufleuchten gebracht wird. Durch diesen Blinklichtbetrieb läßt sich teilweise eine noch höhere Stimulation aufgrund des erhöhten Wahrnehmbarkeits- oder Wechselbehandlungseffekts erzielen.

Nach einer bevorzugten Weiterbildung der Erfindung ist das Farblicht-Behandlungsgerät mit einem Sensor ausgestattet, der einen zeitlich variablen Körperparameter des Anwenders erfaßt. Bei diesem Körperparameter handelt es sich bevorzugt um den Herzschlag. Es ist jedoch auch möglich, als Körperparameter alternativ oder zusätzlich die Hauttemperatur, den elektrischen Hautwiderstand, das EEG, das EKG und/oder den Blutdruck einzusetzen. Auch andere Körperparameter sind möglich, soweit sie sich zeitlich relativ rasch, d.h. innerhalb von Sekunden oder zumindest mehreren Minuten, verändern können und eine Aussage über den Befindungszustand des Körpers beinhalten.

Bei dem erfindungsgemäßen Farblicht-Behandlungsgerät kann die Blinkfrequenz in Abhängigkeit von dem erfaßten Körperparameter gesteuert werden. Dies bringt die erhebliche Verbesserung, daß es nun möglich ist, die Farblichtimpulse mit dem Körperrhythmus zu koppeln. Es stellt sich somit ein Biofeedback-Effekt ein, durch den der Körper positiv beeinflußt werden kann. Die erzielbare therapeutische Wirkung kann hiermit nocht weiter verbessert werden.

Ferner beinhaltet die Kopplung der Blinkfrequenz mit dem gemessenen Körperparameter, insbesondere dem Herzschlag, eine deutliche wahrnehmbare Aussage über den aktuellen Befindungszustand zu erhalten. Durch die Bio-Rückkopplung ist es ferner auch nun leichter möglich, den aktuellen Befindungszustand aktiv positiv durch psychische Konzentration zu beeinflussen und zu verschieben, da beispielsweise jede erreichte Herzschlagfrequenzsenkung sofort über die entsprechende Erniedrigung der Blinkfrequenz signalisiert und hierbei zugleich auch stabilisiert wird. Die Rückkopplung erlaubt somit auch eine Erhöhung der Konzentrationsfähigkeit bei der versuchten Steuerung der Körperparameter im Hinblick auf eine Erhöhung des Wohlbefindens.

Im Falle der Verwendung des Herzschlags als steuernde Körperparameter kann die Herzschlagfrequenz direkt als Blinkfrequenz eingesetzt werden. Alternativ ist es aber auch möglich, einen Frequenzteiler oder Frequenzvervielfacher einzusetzen, um die Blinkfrequenz gegenüber der Herzschlagfrequenz zu erniedrigen oder zu erhöhen, wobei jedoch die Frequenzabhängigkeit erhalten bleibt.

Gleiches gilt auch für die Verwendung eines anderen Körperparameters, beispielsweise des EKG's oder EEG's. Beim Einsatz eines anderen Körperparameters, der sich nicht mit rascher Frequenz verändert, wie beispielsweise bei der Steuerung durch die Hauttemperatur, ist es möglich, jedem Hauttemperaturwert eine bestimmte Blinkfrequenz zuzuordnen, so daß bei Hauttemperaturverschiebungen eine entsprechende Verschiebung der Blinkfrequenz resultiert. Diese Blinkfrequenz kann sich bei Erhöhungen der Hauttemperatur erniedrigen, um zu signalisieren, daß sich der Zustand des Wohlbefindens erhöht hat und der Körper in einen entsprechend ruhigeren Rhythmus gelangt ist.

Eine besonders einfache Erfassung des Körperparameters ohne Notwendigkeit eines invasiven oder belastenden Eingriffs ist bei Verwendung eines Ohrläppchensensors möglich, der die Herzschlagfrequenz über die Veränderung der optischen Gewebedichte im Ohrläppchenbereich mißt. Dieser Ohrläppchensensor kann in nicht belastender, einfacher Weise an das Ohrläppchen angeklemmt werden und liefert die Steuersignale, mit deren Hilfe die Steuereinrichtung die Blinkfrequenz der Behandlungslicht-Strahlungsquelle steuert.

Das Gerät kann als großvolumige und großflächige Lichtbank ausgebildet sein. Für den praktischen, mobilen Einsatz ist es jedoch bevorzugt, das Behandlungsgerät als Handgerät auszugestalten, das über ein Stromkabel an eine herkömmliche Netzsteckdose angeschlossen werden kann. Damit ist das Behandlungsgerät problemlos transportabel und kann an jeden gewünschten Einsatzort benutzt werden.

In bevorzugter Ausgestaltung ist das Handgerät ausschließlich als Niederspannungsgerät ausgelegt, das über ein in das Stromkabel oder den Stecker eingegliedertes Netzteil gespeist wird. Der mit dem Anwender in Berühung kommende Handgerätabschnitt ist somit völlig netzspannungsfrei und wird ausschließlich mit Niederspannung betrieben. Damit ist die elektrische Handhabungssicherheit sehr hoch.

Um einen Betrieb des Behandlungsgeräts auch unabhängig von dem Sensor zu ermöglichen, ist das Gerät vorzugsweise mit einem oder mehreren zusätzlichen manuellen Schaltern versehen, über die das Gerät auf Dauerlichtbetrieb oder auf Blinklichtbetrieb mit fester Blinkfrequenz umschaltbar ist. Es ist auch möglich, verschiedene Blinkfrequenzen vorzusehen, zwischen denen selektiv umgeschaltet werden kann. Diese Umschaltung kann in einfachster Weise über einen einzigen Schalter und einen Mikroprozessor erfolgen, der die Anzahl der Betätigungen des Schalters mißt und eine der Betätigungsanzahl zugeordnete Blinkfrequenz auswählt. Weiterhin kann eine Betriebsart vorgesehen werden, bei der der Blinklichtbetrieb derart steuerbar ist, daß die Intensität des Lichts mit einstellbarer Frequenz und Steigung an- und abschwillt, so daß der Lichtverlauf relativ "weich" ist. Auch diese Betriebsart kann über den Mikroprozessor ausgewertet und herbeigeführt werden. Jeder Betriebsart kann ein eigener Schalter zugeordnet werden.

Der Sensor kann fest mit dem Behandlungsgerät verbunden sein. In bevorzugter Ausgestaltung ist er aber lösbar mit dem Behandlungsgerät gekoppelt, insbesondere über eine Stecker/Kupplungs-Verbindung. Damit kann der Sensor in einfacher Weise durch einen anders gearteten Sensor oder durch einen neuen Sensor ersetzt werden bzw. auch unabhängig vom Handgerät gehandhabt werden.

In diesem Fall überprüft die Steuereinrichtung bevorzugt aktiv, ob der Sensor angekoppelt ist oder nicht, und schaltet lediglich bei eingestecktem Sensor automatisch auf die Betriebsart mit Steuerung der Blinkfrequenz in Abhängigkeit von dem erfaßten Körperparameter um. Wenn der Sensor nicht eingesteckt ist, kann die Steuereinrichtung statt dessen Dauerlichtbetrieb oder feste Blinkfrequenz wählen.

In bevorzugter Ausgestaltung deckt die Behandlungslicht-Strahlungsquelle mehrere separate, schmale Behandlungslicht-Wellenlängenbereiche ab, die durch eine Schalteinrichtung selektiv oder in gewünschter Kombination wählbar sind.

Eine sehr farbreine und technisch einfach realisierbare Lichterzeugung mit hoher Leuchtstärke läßt sich durch Einsatz von Farbleuchtdioden erreichen.

Die Leuchtelemente können in einem festen orthogonalen Raster angeordnet sein. Bevorzugt sind sie aber entlang gebogener Linien angeordnet, so daß beim Aufleuchten der Leuchtelemente sich gekrümmte Leuchtlinien bilden, deren optische Reizwirkung beruhigender wirken kann als ein orthogonales Raster.

In bevorzugter Ausgestaltung sind Leuchtdioden gleicher Wellenlänge jeweils zu Gruppen zusammengefaßt, so daß bei Auswahl der jeweiligen Wellenlängenbereiche die jeweils zugehörige Leuchtdioden-Gruppe mit engem gegenseitigen räumlichen Abstand zum Aufleuchten gebracht wird, so daß sich konzentrierte, hohe Lichtstärke hoher Farbreinheit ergibt.

Die während der Behandlung dem Anwender zugewandte Vorderseite der Behandlungslicht-Strahlungsquelle kann im Normalfall mit einer flachen durchsichtigen Kunststoffplatte abgedeckt sein. Um jedoch eine konzentriertere, punktförmigere Strahlung der Haut und auch eine Durchführung des Lichts durch zwischenliegende, lichtsperrende Kopfhaarbereiche oder dergleichen zu ermöglichen, kann die Vorderseite der Behandlungslicht-Strahlungsquelle auch mit einem Aufsatz versehen sein, der mehrere Vorsprünge aus durchsichtigem Material besitzt, durch die Behandlungslicht bis zu den Spitzen der Vorsprünge geführt werden kann. Diese Vorsprünge können matrixförmig in Form einer Bürste angeordnet sein, so daß das Behandlungsgerät mit dem Aufsatz zum Beispiel auf den Hinterkopf aufgesetzt werden kann und die Vorsprünge durch die Haare hindurch bis zur Kopfhaut reichen. Das Behandlungslicht kann somit durch diese Vorsprünge zwischen den Haaren hindurch bis zur Kopfhaut gelangen und dort die gewünschte therapeutische Wirkung erzielen.

Die Vorsprünge sitzen bevorzugt mittig über dem jeweils zugehörigen Leuchtelement, so daß das von diesem abgegebene Farblicht mittig in den Vorsprung hineingeführt wird und somit mit hohem Wirkungsgrad durch diesen hindurch bis zur Spitze des Vorsprungs geleitet und dann aus dieser Spitze austreten kann.

Bei konischem Zulaufen dieser Spitzen ist die Durchdringung der störenden, zwischenliegenden Bereiche, insbesondere des Kopfhaars, noch weiter erleichtert. Zudem kann das Licht sehr stark gebündelt und auf kleine Punkte konzentriert werden und somit dort sehr konzentriert auf die Körperhaut auftreffen.

In bevorzugter Ausgestaltung ist das als Handgerät ausgestaltete Farblicht-Behandlungsgerät mit langgestreckter Gestalt versehen und besitzt unterschiedliche Breite. Der schmalere Bereich, aus dem vorzugsweise auch das Stromversorgungskabel austritt und in dem der Sensor eingesteckt werden kann, dient als Handgriff, so daß das Farblicht-Behandlungsgerät sehr einfach gehandhabt und zuverlässig und stabil ergriffen werden kann. Der Kopfteil besitzt demgegenüber größere Breite und ermöglicht somit die Unterbringung einer Mehrzahl von Farbleuchtdioden. Die Unterteilung des Behandlungsgeräts in Handgriff und Bestrahlungsfeld stellt zugleich auch sicher, daß keine unbeabsichtigte Abdeckung einzelner Leuchtdioden durch die Hand während der Bestrahlung stattfindet. Das erfindungsgemäße Gerät ermöglicht somit die Erzielung einer wirksamen therapeutischen Wirkung, d.h. ist ein Farblicht-Therapiegerät, das eine oder mehrere reine Farben erzeugen kann.

Gemäß einem noch weiteren Gesichtspunkt der Erfindung kann das Farblicht-Behandlungsgerät mit Reizstrom-Elektroden versehen sein, mittels denen die Haut eines Anwenders mit Reizstrom beaufschlagt werden kann. Die Vorteile solcher Reizstrom-Elektroden sind bekannt und brauchen daher nicht näher erläutert zu werden. Vorzugsweise erzeugen die Reizstrom-Elektroden einen gepulsten Strom, dessen Frequenz und/oder Stromstärke ggf. einstellbar oder veränderbar ist. Die Reizstrom-Elektroden können an der gleichen Seite des Gerätegehäuses wie die Behandlungslicht-Strahlungsquelle angebracht sein, und zwar vorzugsweise etwa im Zentrum der die Behandlungslicht-Strahlungsquelle aufweisenden Seite des Gerätegehäuses.

Nach einer noch anderen Weiterbildung der Erfindung kann das Farblicht-Behandlungsgerät zusätzlich einen Ultraschallwandler aufweisen, mittels dem Präparate oder dergleichen in die Hautoberfläche eines Anwenders eingeschleust werden können. Dieser Ultraschallwandler wird vorzugsweise mit einer festen Betriebsfrequenz von ca. 25kHz betrieben und enthält z.B. ein Piezoelement. Auch der Ultraschallwandler wird vorzugsweise an der gleichen Seite des Gerätegehäuses wie die Behandlungslicht-Strahlungsquelle und vorzugsweise etwa in deren Mitte angebracht.

Schließlich schlägt die Erfindung auch ein Farblicht-Behandlungsgerät vor, bei dem zusätzlich ein Duftgenerator vorgesehen ist, mittels dem ein wohlriechender Duft erzeugt werden kann. In den Duftgenerator können vorzugsweise Duftkarten (Aromakarten) oder Duftchips (Aromachips) eingeführt werden, die jeweils unterschiedliche Duftrichtungen erzeugen, so daß sich jeder Anwender den liebsten Duft aussuchen kann. Der Duftgenerator verströmt die in den Aromakarten oder Aromachips enthaltenen ätherischen Öle, wobei hierzu vorzugsweise die die von dem Trafo und/oder der Steuereinrichtung erzeugte Abwärme ausgenutzt wird, so daß eine äußerst effiziente Energienutzung erzielt wird.

Im Falle der vorstehend genannten Zusatzeinrichtungen (Reizstrom-Elektroden, Ultraschallwandler, Duftgenerator) ist es zweckmäßig, am Gerätegehäuse einen jeweiligen, mit der Steuereinrichtung verbundenen Schalter vorzusehen, der die betreffende Zusatzeinrichtung bei seiner Betätigung, ggf. mittels des Mikroprozessors, aktiviert.

Selbstverständlich ist es auch möglich, bei Bedarf jede Kombination aus den genannten Zusatzeinrichtungen (Reizstrom-Elektroden und/oder Ultraschallwandler und/oder Duftgenerator) am erfindungsgemäßen Farblicht-Behandlungsgerät vorzusehen.

Die Erfindung wird nachstehend anhand eines Ausführungsbeispiels unter Bezugnahme auf die Zeichnungen näher beschrieben. Es zeigen:
**Fig.1** eine schematische Darstellung eines Ausführungsbeispiels des Farblicht-Behandlungsgeräts,
**Fig.2** die Bestückung des Kopfteils des Behandlungsgeräts mit Leuchtdioden,
**Fig.3** eine schematische Darstellung der Anordnung der Schalter-, Steuer- und Kontaktelemente auf einer Leiterplatine im Behandlungsgerät und
**Fig.4** eine Darstellung der elektrischen Verschaltung der Leuchtdioden.

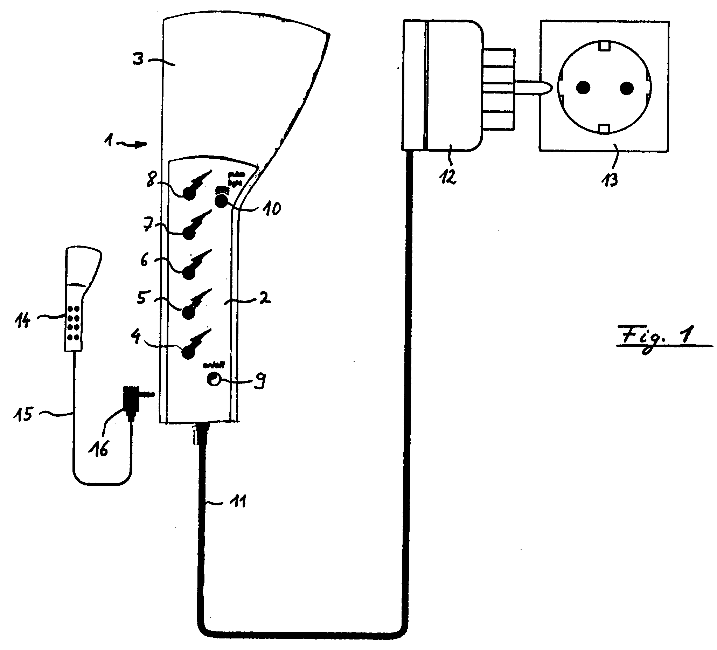

Das in Fig.1 gezeigte Ausführungsbeispiel des Farblicht-Behandlungsgeräts weist ein Handgerät 1 auf, das aus einem Griffteil 2 und einem integral in den Griffteil 2 übergehenden Kopfteil 3 zusammengesetzt ist. Der Griffteil 2 ist relativ schmal gehalten, so daß er leicht mit der Hand umgriffen werden kann, und trägt Farbwahlschalter 4 bis 8, einen Ein/Aus-Schalter 9 und einen Schalter 10 zur Auswahl des Blinklichtbetriebs der Farblichterzeugung sowie gegebenenfalls zur Einstellung der (jeweils festen) Blinkfrequenz. Über die Farbwahlschalter 4 bis 8 läßt sich die jeweils gewünschte Farbe des Farblichts wählen, nämlich grün, gelb, orange, rot und blau. Durch die Betätigung des Ein/Aus-Schalters 9 wird das Gerät ein- und ausgeschaltet. Bei Einschaltung des Geräts und nicht eingestecktem Sensor befindet sich das Gerät im Dauerlichtbetrieb, d.h. gibt kontinuierlich die über den jeweils betätigten Farbwahlschalter 4 bis 8 ausgewählte Farbe oder auch eine aus mehreren Farben zusammengesetzte Mischfarbe ab. Bei Betätigung des Schalters 10 wird auf Blinklichtbetrieb mit fester Blinkfrequenz übergegangen, wobei gegebenenfalls auch zwischen mehreren festen Blinkfrequenzen ausgewählt werden kann, zum Beispiel durch entsprechend häufige Betätigung des Schalters 10.

Es kann auch ein (nicht gezeigter) Schalter vorgesehen werden, bei dessen Betätigung ein solcher Blinklichtbetrieb hervorgerufen wird, bei dem die Intensität des Lichts mit einstellbarer Frequenz und Steigung an- und abschwillt; so daß der Lichtverlauf relativ "weich" ist. Auch diese Betriebsart kann ggf. durch eine entsprechend häufige Betätigung des Schalters 10 herbeigeführt werden.

In Fig. 1 ist das Handgerät 1 von der Rückseite gezeigt, so daß die im Kopfteil 3 angeordneten Spezialleuchtdioden, die sehr reine Farben mit schmalem Wellenlängenbereich emittieren, nicht sichtbar sind.

Das Handgerät 1 ist mit einem Stromkabel 11 verbunden, das an der Stirnseite des Griffteils 2 in das Handgerät eingeführt ist. Am anderen Ende des Stromkabels 11 ist ein Steckernetzteil 12 angebracht, das eine lösbare Verbindung mit einer üblichen Haushaltssteckdose 13 ermöglicht. Das Steckernetzteil 12 transformiert die übliche Netzspannung von 220 V/230 V auf einen Niederspannungswert, beispielsweise 5 V herab, so daß das Handgerät 1 ausschließlich mit niedriger Spannung gespeist wird und auch im Stromkabel 11 lediglich Niederspannung auftritt. Die elektrische Sicherheit des Handgeräts ist damit sehr hoch.

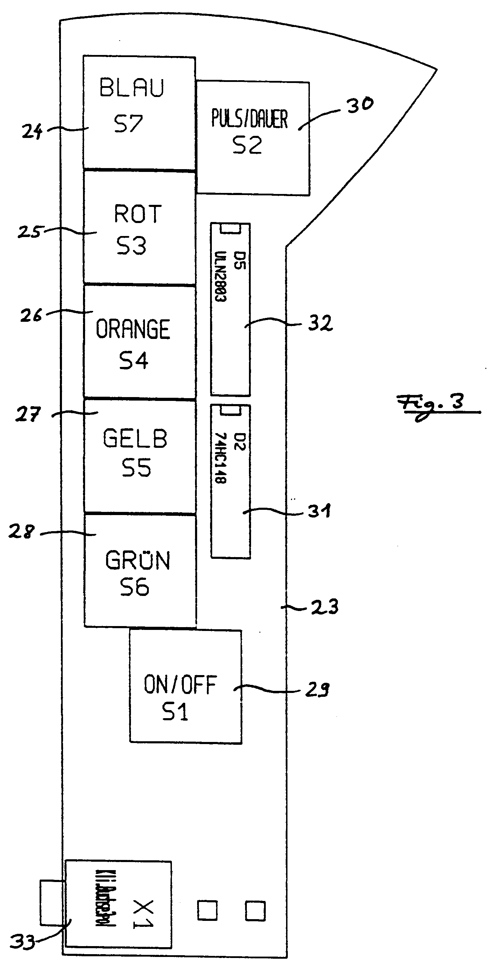

Das Handgerät 1 ist mit einer Aufnahmevorrichtung, vorzugsweise in der Form einer in Fig. 3 gezeigten Klinkenbuchse 33 versehen, die seitlich am Griffteil 2 in dessen unteren Bereich nahe beim Eintritt des Stromkabels 11 angeordnet ist. Die Klinkenbuchse ist bevorzugt als dreipolige Klinkenbuchse ausgelegt und dient zur Kopplung des Handgeräts 1 mit einem Sensor 14 zur Erfassung eines Körperparameters, der bei dem dargestellten Ausführungsbeispiel als Ohrläppchen-Infrarot-Sensor ausgelegt ist und den Herzschlag des Anwenders, an dessen Ohrläppchen er befestigt wird, erfaßt. Der Sensor 14 ist über ein Kabel 15 mit einem Klinkenstecker 16 verbunden, der entsprechend der Klinkenbuchse als dreipoliger Stecker ausgelegt und in diese einsteckbar ist. Der Sensor 14 gibt somit dann, wenn er an das Ohrläppchen des Anwenders angesetzt ist, ein Signal an das Handgerät 1 und die in diesem enthaltene Steuereinrichtung ab, das über seine Amplitudenveränderungen den Herzschlag bzw. den Puls des Anwenders repräsentiert. Die Steuereinrichtung ist so ausgelegt, daß sie bei eingeschaltetem Handgerät 1 und angestecktem Sensor 14 die Amplitudenänderungen des Sensor-Ausgangssignals auswertet und die jeweils ausgewählte Farbleuchtdioden-Gruppe mit der Frequenz des Herzschlags ein- und ausschaltet, so daß die ausgewählte Leuchtdiodengruppe im Rhythmus des Herzschlags blinkt. Um diese Steuerungsfunktion zu erreichen, kann die Steuereinrichtung beispielsweise die ansteigenden und abfallenden Flanken des Sensor-Ausgangssignals (zum Beispiel über Differenzierung) erfassen und die Leuchtdioden bei ansteigender Flanke einschalten und bei abfallender Flanke wieder ausschalten. Die Ausgestaltung kann auch derart getroffen sein, daß zwischen die Steuereinrichtung und den Leuchtdiodentreiber ein Schaltglied geschaltet ist, das durch das Sensor-Ausgangssignal ein- und ausgeschaltet wird (und sich beispielsweise lediglich oberhalb eines bestimmten Amplitudenwerts des Sensor-Ausgangssignals im Ein- oder Ausschaltzustand befindet) und somit die Durchleitung des von der Steuereinrichtung kontinuierlich erzeugten Aktivierungssignals jeweils während gewisser Phasen des Sensor-Ausgangssignals unterbricht.

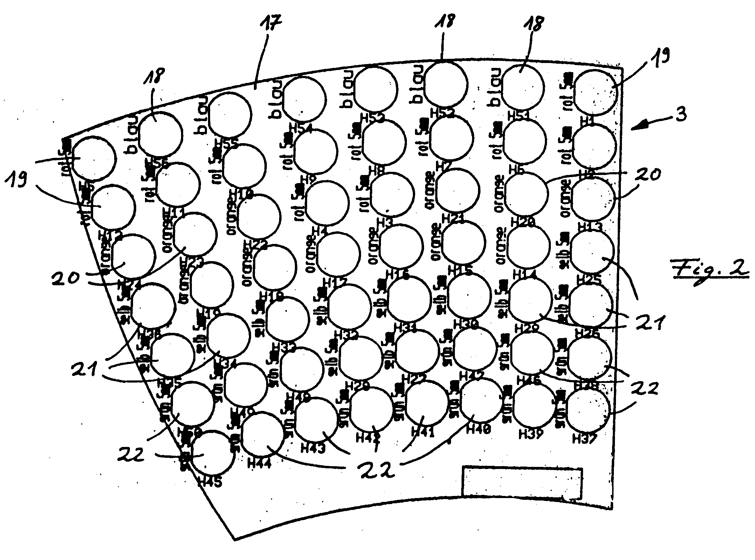

In Fig. 2 ist eine Draufsicht auf die Leuchtdiodenanordnung im Kopfteil 3 dargestellt. Die Leuchtdiodenanordnung bildet die Behandlungslicht-Strahlungsquelle und ist auf einer Leiterplatine 17 montiert, über die die einzelnen Leuchtdioden mechanisch gehalten und elektrisch kontaktiert werden. Anstelle von Leuchtdioden können auch andere Leuchtelemente wie Laserdioden oder sonstige lichterzeugende Elemente eingesetzt werden, vorausgesetzt, daß diese imstande sind, jeweils farbiges Licht mit engen Wellenlängenbereichen zu erzeugen. Bevorzugt sind jedoch Spezialleuchtdioden, da diese hohe Lichtstärke bei insgesamt verhältnismäßig niedrigem Energiebedarf und hoher Farbreinheit erzeugen können.

Bei dem gezeigten Ausführungsbeispiel besteht die Leuchtdiodenanordnung aus mehreren Gruppen von Leuchtdioden jeweils unterschiedlicher Farbe. Bei dem gezeigten Ausführungsbeispiel ist eine Gruppe von blauen Leuchtdioden 18, eine Gruppe von roten Leuchtdioden 19, eine Gruppe von orangen Leuchtdioden 20, eine Gruppe von gelben Leuchtdioden 21 und eine Gruppe von grünen Leuchtdioden 22 vorhanden. Aus Gründen der Übersichtlichkeit sind nicht alle Leuchtdioden jeweils mit einem Bezugszeichen versehen, tragen aber jeweils die zugehörige Farbbezeichnung.

Gemäß Fig. 2 sind die zu einer jeweiligen Gruppe gehörenden Leuchtdioden jeweils zusammengefaßt, so daß sich bei Ansteuerung einer jeweiligen Gruppe ein intensiv leuchtender Farbstreifen ohne größere streifeninterne Intensitätsschwankungen (mit Ausnahme der naturgegebenen Intensitätsschwankungen aufgrund des gegenseitigen räumlichen Abstands der Leuchtdioden der Gruppe) ergibt. Weiterhin sind die Leuchtdioden einer jeweiligen Gruppe zumindest größtenteils entlang einer oder zwei gebogenen Linien angeordnet, so daß die bei Erregung einer jeweiligen Leuchtdiodengruppe sich einstellenden Farbstreifen gekrümmt verlaufen, wie aus der Fig. 2 ersichtlich ist. In radialer Richtung sind die Leuchtdioden der Leuchtdiodenanordnung allerdings auf im wesentlichen radialverlaufenden geradlinigen Linien angeordnet.

Die in Fig. 2 gezeigte Leiterplatine 17 mit den Leuchtdioden 18 bis 22 ist durch eine lichtdurchlässige Abdeckung, vorzugsweise aus völlig transparentem Kunststoff, abgedeckt, so daß das beim Betrieb des Handgeräts 1 erzeugte Farblicht ungefiltert und ungedämpft auf den Anwender einwirken kann.

In Fig. 3 ist in Form eines Blockschaltbilds die elektrische Schaltung des Ausführungsbeispiels des Farblicht-Behandlungsgeräts dargestellt. Auf einer Leiterplatine 23, die im Innern des Griffteils 2 angeordnet ist und mit ihrer oberen, sich verbreiternden Seite geringfügig in den Kopfteil 3 hineinragt, sind Schaltelemente 24 bis 28 angeordnet, die durch die in Fig. 1 gezeigten Schalter 4 bis 8 betätigt werden können und zur Ein- und Ausschaltung der blauen, roten, orangen, gelben bzw. grünen Leuchtdiodengruppe (siehe Fig. 2) dienen. Weiterhin ist auf der Schaltplatine 23 ein Ein/Aus-Schaltelement 28, das durch den in Fig.1 gezeigten Schalter 9 betätigt wird, und ein Puls/Dauer-Umschalter 30 angeordnet, der durch den in Fig. 1 gezeigten Schalter 10 betätigt wird. Weiterhin ist auf der Schaltplatine 23 eine Steuereinrichtung 31 angeordnet, die vorzugsweise als Mikroprozessor ausgestaltet ist und die Schaltzustände der Schalter 24 bis 29 abfragt und die Ein- bzw. Ausschaltung des gesamten Handgeräts bzw. die Ein- bzw. Ausschaltung der jeweils auszuwählenden Leuchtdiodengruppe steuert. Die Steuereinrichtung 31 kann auch mit dem Puls/Dauer-Schalter 30 verbunden sein und bei einer jeweiligen Betätigung dieses Schalters 30 zwischen Dauerlichtbetrieb und Blinklichtbetrieb umschalten. Alternativ kann der Puls/Dauer-Schalter 30 auch mit einem eigenen Steuerelement, beispielsweise in Form eines Zählers verbunden sein, der die Anzahl der aufeinanderfolgenden Betätigungen des Schalters 30 zählt und die jeweilige Blinkfrequenz der Leuchtdiodenerregung in Abhängigkeit vom Zählstand festlegt. Hierbei kann eine nicht gezeigte, einen separaten Bestandteil der Steuereinrichtung bildende Zwischenstufe vorhanden sein, die das Ausgangssignal der Steuereinrichtung 31 an eine Treiberschaltung 32 für die Generierung des Leuchtdiodenstroms und der Leuchtdiodenspannung weiterleitet. Das Ausgangssignal der mit dem Puls/Dauer-Schalter 30 verbundenen Steuerstufe kann hierbei in gleicher Weise wie das Ausgangssignal des Sensors 14 an den Aktivierungseingang dieser Zwischenstufe angelegt werden, so daß das Ausgangssignal der Steuereinrichtung 31 lediglich im Rhythmus des Ausgangssignals des Ausgangssignals der Steuerstufe bzw. - bei angeschlossenem Sensor 14 - im Rhythmus des Ausgangssignals des Sensors 14 an die Treibereinrichtung 32 weitergeleitet wird, so daß die Treibereinrichtung 32 die notwendige Leuchtdiodenspannung und den notwendigen Leuchtdiodenstrom lediglich im Rhythmus des Ausgangssignals der Zwischenstufe generiert. Damit blinkt die jeweils ausgewählte Leuchtdiodengruppe im gewünschten Rhythmus entweder des durch den Sensor 14 erfaßten Körperparameters oder - bei gewünschter fester Blinkfrequenz - mit der ausgewählten Frequenz.

Auf der Leiterplatine 23 ist ferner eine 3-polige Klinkenbuchse 33 vorhanden, in die - über eine entsprechende Öffnung im Gehäuse des Handgeräts 1 - der Stecker 16 des Sensors 14 einsteckbar ist. Die Steuereinrichtung 31 kann so ausgelegt sein, daß sie das Einstecken bzw. Eingestecktseins des Steckers 16 in die Klinkenbuchse 33 erfaßt und auf die Betriebsart der Blinkfrequenzsteuerung in Abhängigkeit vom Ausgangssignal des Sensors 14 umschaltet. Alternativ kann das Ausgangssignal des Sensors 14 durch eine entsprechende Wandlerschaltung, beispielsweise einen Impulsformer, in ein Impulssignal mit der Frequenz des Sensor-Ausgangssignals umgewandelt werden, das an einem Aktivierungseingang einer der Steuereinrichtung 31 nachgeschalteten Zwischenstufe anliegt.

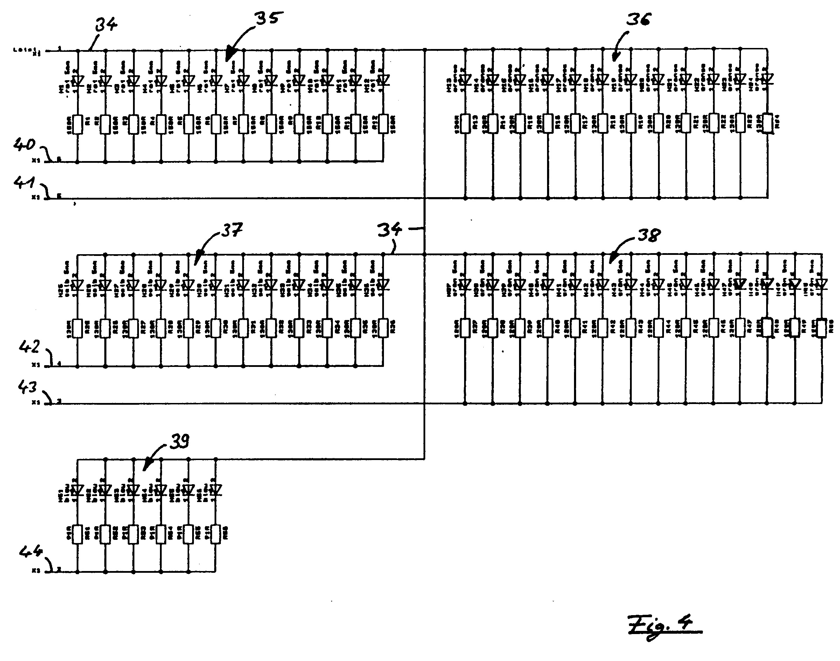

In Fig. 4 ist die elektrische Verschaltung der Leuchtdioden 18 bis 22 (Fig. 2) in größeren Einzelheiten gezeigt. Alle Leuchtdioden sind in Matrixanordnung verschaltet, wobei alle Leuchtdioden durch eine gemeinsame Speisespannungsleitung 34 mit positiver Spannung gespeist werden, wenn das Handgerät 1 eingeschaltet ist. Die Leuchtdioden sind jeweils gruppenweise zusammengefaßt, wobei die Leuchtdiodengruppe 35 den roten Leuchtdioden 19, die Leuchtdiodengruppe 36 den orangen Leuchtdioden 20, die Leuchtdiodengruppe 37 den gelben Leuchtdioden 21, die Leuchtdiodengruppe 38 den grünen Leuchtdioden 22 und die Leuchtdiodengruppe 39 den blauen Leuchtdioden 18 entspricht. Die Auswahl einer jeweiligen Leuchtdiodengruppe erfolgt durch Anlegen eines Wählsignals an einen der Wählanschlüsse 40 bis 44. Das jeweilige Wählsignal wird in Abhängigkeit von dem jeweils betätigten Schalter 4 bis 8 durch die Steuereinrichtung 31 über die Treibereinrichtung 32 an die jeweilige Leuchtdiodengruppe 35 bis 39 angelegt. Durch die gezeigte Verschaltung ist sichergestellt, daß alle Leuchtdioden einer jeweiligen Leuchtdiodengruppe 35 bis 39 jeweils mit gleicher Intensität leuchten und das Handgerät selbst dann noch funktionsfähig ist, wenn einzelne Leuchtdioden aufgrund von Fehlern oder Alterung oder dergleichen nicht länger funktionsfähig sein sollten. Dies liegt in der Parallelschaltung der einzelnen Leuchtdioden einer jeweiligen Leuchtdiodengruppe begründet.

In einer (nicht dargestellten) Ausführungsform der Erfindung weist das Farblicht-Behandlungsgerät zusätzlich Reizstrom-Elektroden auf, mittels denen die Haut eines Anwenders mit Reizstrom beaufschlagt werden kann. Derartige Reizstrom-Elektroden und deren Anwednungsgebiete sowie Vorteile sind an sich bekannt, so daß auf eine nähere Erläuterung an dieser Stelle verzichtet werden kann. Vorzugsweise erzeugen die Reizstrom-Elektroden einen gepulsten Strom, dessen Frequenz und/oder Stromstärke ggf. einstellbar oder veränderbar ist. Die Reizstrom-Elektroden sind vorzugsweise an der gleichen Seite des Gerätegehäuses wie die Behandlungslicht-Strahlungsquelle angebracht, und zwar vorzugsweise etwa im Zentrum der die Behandlungslicht-Strahlungsquelle aufweisenden Seite des Gerätegehäuses.

Nach einer noch anderen (nicht gezeigten) Ausführungsform der Erfindung kann das Farblicht-Behandlungsgerät zusätzlich einen Ultraschallwandler aufweisen, mittels dem Präparate oder dergleichen in die Hautoberfläche eines Anwenders eingeschleust werden können. Dieser Ultraschallwandler wird vorzugsweise mit einer festen Betriebsfrequenz von ca. 25kHz betrieben und enthält z.B. ein Piezoelement. Auch der Ultraschallwandler wird vorzugsweise an der gleichen Seite des Gerätegehäuses wie die Behandlungslicht-Strahlungsquelle und vorzugsweise etwa in deren Mitte angebracht.

Ein weitere Variante des Farblicht-Behandlungsgeräts sieht zusätzlich einen Duftgenerator vor, mittels dem ein wohlriechender Duft erzeugt werden kann. In den Duftgenerator können vorzugsweise Duftkarten (Aromakarten) oder Duftchips (Aromachips) eingeführt werden, die jeweils unterschiedliche Duftrichtungen erzeugen, so daß sich jeder Anwender den jeweils liebsten Duft aussuchen kann. Der Duftgenerator verströmt die in den Aromakarten oder Aromachips enthaltenen ätherischen Öle, wobei hierzu vorzugsweise die die von dem Trafo und/oder der Steuereinrichtung erzeugte Abwärme ausgenutzt wird, so daß eine äußerst effiziente Energienutzung erzielt wird.

Im Falle der vorstehend genannten Zusatzeinrichtungen (Reizstrom-Elektroden, Ultraschallwandler, Duftgenerator) ist es zweckmäßig, am Gerätegehäuse einen jeweiligen, mit der Steuereinrichtung verbundenen Schalter vorzusehen, der die betreffende Zusatzeinrichtung bei seiner Betätigung, ggf. mittels des Mikroprozessors, aktiviert.

Selbstverständlich ist es möglich, bei Bedarf jede Kombination aus den genannten Zusatzeinrichtungen (Reizstrom-Elektroden und/oder Ultraschallwandler und/oder Duftgenerator) am erfindungsgemäßen Farblicht-Behandlungsgerät vorzusehen. Diese können ggf. mittels eines einzigen Schalters durch eine entsprechende Bedienungsfolge angesteuert bzw. aktiviert werden. Somit kann ein äußerst vielseitig verwendbares Kombinationsgerät geschaffen werden.

## Patentansprüche

1. Farblicht-Behandlungsgerät mit einer Farblicht-Strahlungsquelle (18 bis 22), *dadurch gekennzeichnet, daß* die Farblicht-Strahlungsquelle (18 bis 22) mittels einer Steuereinrichtung (31, 32) in einen Blinklichtbetrieb mit einer bestimmten Blinkfrequenz versetzbar ist.

2. Farblicht-Behandlungsgerät nach Anspruch 1, *gekennzeichnet durch* einen Sensor (14 bis 16) zur Erfassung eines sich zeitlich verändernden Körperparameters des Benutzers oder Anwenders des Farblicht-Behandlungsgeräts, wobei der Sensor (14 bis 16) mit der Steuereinrichtung zur Steuerung der Blinkfrequenz der Farblicht-Strahlungsquelle in Abhängigkeit vom Körperparameter koppelbar ist.

3. Farblicht-Behandlungsgerät nach Anspruch 2, *dadurch gekennzeichnet,* daß der Körperparameter der Herzschlag ist.

4. Farblicht-Behandlungsgerät nach Anspruch 2 oder 3, *dadurch gekennzeichnet,* daß der Körperparameter die Hauttemperatur, der elektrische Hautwiderstand, das EEG, das EKG und/oder der Blutdruck ist.

5. Farblicht-Behandlungsgerät nach einem der Ansprüche 2 bis 4, *dadurch gekennzeichnet,* daß der Sensor (14 bis 16) als Ohrläppchen-Sensor, insbesondere als Infrarot-Sensor ausgebildet ist.

6. Farblicht-Behandlungsgerät nach einem der vorhergehenden Ansprüche, *dadurch gekennzeichnet,* daß es als Handgerät (1) mit einem Stromkabel (11) für den Anschluß an einer Standard-Netzfrequenz-Steckdose (13) ausgebildet ist.

7. Farblicht-Behandlungsgerät nach Anspruch 6, *dadurch gekennzeichnet,* daß das Handgerät (1) ein Niederspannungsgerät ist und das Stromkabel (11) mit einem Transformator, vorzugsweise einem Steckernetzteil (12), zur Transformierung der Netzspannung in eine Niederspannung versehen ist.

8. Farblicht-Behandlungsgerät nach einem der vorhergehenden Ansprüche, *dadurch gekennzeichnet,* daß mittels der Steuereinrichtung (31, 32) mehrere verschiedene Betriebsarten des Blinklichtbetriebs realiserbar sind, wobei in einer ersten Betriebsart der Blinklichtbetrieb derart steuerbar ist, daß die Intensität des Lichts mit einstellbarer Frequenz und Steigung an- und abschwillt, und/oder in einer zweiten Betriebsart ein Blinklichtbetrieb mit einer festen, gegebenenfalls variablen Blinkfrequenz einstellbar ist und/oder in einer dritten Betriebsart ein Dauerlichtbetrieb der Farblicht-Strahlungsquelle herbeiführbar ist.

9. Farblicht-Behandlungsgerät nach Anspruch 8, *dadurch gekennzeichnet,* daß am Gerätegehäuse ein oder mehrere mit der Steuereinrichtung (31, 32) verbundene Schalter (4 bis 10) zur Einstellung der gewünschten Betriebsart der Farblicht-Strahlungsquelle angebracht sind.

10. Farblicht-Behandlungsgerät nach einem der Ansprüche 2 bis 9, *dadurch gekennzeichnet,* daß der Sensor (14 bis 16) mit einem Stecker (16) versehen ist und am Behandlungsgerät (1) ein Kupplungsstück (33) für die lösbare Aufnahme des Steckers (16) angebracht ist.

11. Farblicht-Behandlungsgerät nach Anspruch 10, *dadurch gekennzeichnet,* daß die Steuereinrichtung (31, 32) zur Überprüfung, ob der Sensor (14 bis 16) mit dem Behandlungsgerät (1) verbunden ist, und zur Steuerung der Gerätebetriebsart (in seiner Intensität an- und abschwellendes Licht, Licht mit variabler Blinkfrequenz oder fester Blinkfrequenz oder Dauerlichtbetrieb) in Abhängigkeit vom Überprüfungsergebnis ausgelegt ist.

12. Farblicht-Behandlungsgerät nach einem der vorhergehenden Ansprüche, *dadurch gekennzeichnet,* daß die Behandlungslicht-Strahlungsquelle mehrere Farbleuchtelemente (18 bis 22) und eine Schalteinrichtung (4 bis 8, 24 bis 28) zur Auswahl des oder der Wellenlängenbereiche des Behandlungslichts aufweist.

13. Farblicht-Behandlungsgerät nach einem der vorhergehenden Ansprüche, *dadurch gekennzeichnet,* daß die Behandlungslicht-Strahlungsquelle eine Mehrzahl von Farbleuchtdioden (18 bis 22) aufweist, die auf gebogenen Linien angeordnet sind.

14. Farblicht-Behandlungsgerät nach einem der vorhergehenden Ansprüche, *dadurch gekennzeichnet,* daß die jeweils gleichen Wellenlängenbereich aufweisenden Leuchtdioden zu Gruppen (35 bis 39) zusammengefaßt sind.

15. Farblicht-Behandlungsgerät, insbesondere nach einem der vorhergehenden Ansprüche, *dadurch gekennzeichnet,* daß die Behandlungslicht-Strahlungsquelle mit einem Aufsatz aus durchsichtigem Material versehen ist, der mehrere vorstehende Vorsprünge zur Lichtführung besitzt.

16. Farblicht-Behandlungsgerät nach Anspruch 15, *dadurch gekennzeichnet,* daß für jedes lichterzeugende Element (18 bis 22) ein Vorsprung vorgesehen ist, der im wesentlichen mittig über dem zugehörigen lichterzeugenden Element sitzt.

17. Farblicht-Behandlungsgerät nach Anspruch 15 oder 16, *dadurch gekennzeichnet,* daß jeder Vorsprung im wesentlichen konisch zuläuft.

18. Farblicht-Behandlungsgerät, insbesondere nach einem der vorhergehenden Ansprüche, *dadurch gekennzeichnet,* daß das als Handgerät (1) ausgelegte Behandlungsgerät langgestreckte Gestalt mit einem als Griff dienenden schmaleren Bereich (2) und einem im Vergleich hierzu breiteren Kopfbereich (3), in dem die Leuchtdioden der Behandlungslicht-Strahlungsquelle angeordnet sind, besitzt.

19. Farblicht-Behandlungsgerät nach einem der vorhergehenden Ansprüche, *dadurch gekennzeichnet,* daß es ein therapeutisches Farblicht-Behandlungsgerät ist, das zur selektiven und/oder kombinatorischen Erzeugung von Farblicht mit schmalem Wellenlängenbereich ausgelegt ist.

20. Farblicht-Behandlungsgerät, insbesondere nach einem der vorhergehenden Ansprüche, *dadurch gekennzeichnet,* daß Reizstrom-Elektroden vorgesehen sind, mittels denen die Haut eines Anwenders mit Reizstrom beaufschlagbar ist.

21. Farblicht-Behandlungsgerät nach Anspruch 20, *dadurch gekennzeichnet,* daß die Reizstrom-Elektroden einen gepulsten Strom erzeugen, dessen Frequenz und/oder Stromstärke ggf. einstellbar oder veränderbar ist.

22. Farblicht-Behandlungsgerät nach Anspruch 20 oder 21, *dadurch gekennzeichnet,* daß am Gerätegehäuse ein mit der Steuereinrichtung (31, 32) verbundener Schalter zum Aktivieren der Reizstrom-Elektroden und ggf. zum Ändern der Frequenz und/oder Stromstärke angebracht ist.

23. Farblicht-Behandlungsgerät nach einem der Ansprüche 20 bis 22, *dadurch gekennzeichnet,* daß die Reizstrom-Elektroden an der gleichen Seite des Gerätegehäuses wie die Behandlungslicht-Strahlungsquelle angebracht sind.

24. Farblicht-Behandlungsgerät nach einem der Ansprüche 20 bis 23, *dadurch gekennzeichnet,* daß die Reizstrom-Elektroden etwa im Zentrum der die Behandlungslicht-Strahlungsquelle aufweisenden Seite des Gerätegehäuses angebracht sind.

25. Farblicht-Behandlungsgerät, insbesondere nach einem der vorhergehenden Ansprüche, *dadurch gekennzeichnet,* daß ein Ultraschallwandler vorgesehen ist, mittels dem Präparate oder dergleichen in die Hautoberfläche eines Anwenders einschleusbar sind.

26. Farblicht-Behandlungsgerät nach Anspruch 25, *dadurch gekennzeichnet,* daß der Ultraschallwandler mit einer festen Betriebsfrequenz von vorzugsweise ca. 25kHz betrieben wird.

27. Farblicht-Behandlungsgerät nach Anspruch 25 oder 26, *dadurch gekennzeichnet,* daß am Gerätegehäuse ein mit der Steuereinrichtung (31, 32) verbundener Schalter zum Aktivieren des Ultraschallwandlers angebracht ist.

28. Farblicht-Behandlungsgerät nach einem der Ansprüche 25 bis 27, *dadurch gekennzeichnet,* daß der Ultraschallwandler an der gleichen Seite des Gerätegehäuses wie die Behandlungslicht-Strahlungsquelle und vorzugsweise etwa in deren Mitte angebracht ist.

29. Farblicht-Behandlungsgerät nach einem der Ansprüche 25 bis 28, *dadurch gekennzeichnet,* daß der Ultraschallwandler ein Piezoelement enthält.

30. Farblicht-Behandlungsgerät, insbesondere nach einem der vorhergehenden Ansprüche, *dadurch gekennzeichnet,* daß ein Duftgenerator vorgesehen ist, mittels dem ein wohlriechender Duft erzeugbar ist.

31. Farblicht-Behandlungsgerät nach Anspruch 30, *dadurch gekennzeichnet,* daß in den Duftgenerator Duftkarten oder Duftchips einführbar sind, die jeweils unterschiedliche Duftrichtungen erzeugen.

32. Farblicht-Behandlungsgerät nach Anspruch 30 oder 31, *dadurch gekennzeichnet,* daß in den Duftgenerator ätherische Öle verströmt.

33. Farblicht-Behandlungsgerät nach Anspruch 32, *dadurch gekennzeichnet,* daß der Duftgenerator zum Verströmen der ätherischen Öle die von dem Trafo und/oder der Steuereinrichtung (31, 32) erzeugte Abwärme verwendet.

34. Farblicht-Behandlungsgerät nach einem der Ansprüche 30 bis 33, *dadurch gekennzeichnet,* daß am Gerätegehäuse ein mit der Steuereinrichtung (31, 32) verbundener Schalter zum Aktivieren des Duftgenerators angebracht ist.

35. Farblicht-Behandlungsgerät, *gekennzeichnet durch* Reizstrom-Elektroden nach einem der Ansprüche 20 bis 24 und/oder einen Ultraschallwandler nach einem der Ansprüche 25 bis 29 und/oder einen Duftgenerator nach einem der Ansprüche 30 bis 34.
